# EUROPEAN PATENT SPECIFICATION

(11) **EP 2 451 787 B1**
(45) Date of publication and mention of the grant of the patent: **24.04.2013**
(21) Application number: 10729777.2
(22) Date of filing: 01.07.2010
(51) Int. Cl.: C07D 239/48, A61K 31/505, A61P 11/06

(54) **POLYMORPH OF [4,6-BIS(DIMETHYLAMINO)-2-(4-{[4-(TRIFLUOROMETHYL)BENZOYL]AMINO}BENZYL)PYRIMIDIN-5-YL]**
POLYMORPH VON [4,6-BIS(DIMETHYLAMINO)-2-(4-{[4-(TRIFLUORMETHYL)BENZOYL]AMINO}BENZYL)PYRIMIDIN-5-YL]
POLYMORPHE DE [4,6-BIS(DIMÉTHYLAMINO)-2-(4-{[4-(TRIFLUOROMÉTHYL)BENZOYL]AMINO}BENZYL)PYRIMIDIN-5-YL]ACÉTIQUE

(30) Priority: 06.07.2009 US 223165 P
(43) Date of publication of application: 16.05.2012
(73) Proprietor: Boehringer Ingelheim International GmbH, 55216 Ingelheim (DE)
(72) Inventor: KIM, Soojin, Ridgefield, Connecticut 06877-0368 (US)
(74) Representative: Simon, Elke Anna Maria
(86) International application number: PCT/US2010/040733
(87) International publication number: WO 2011/005645

(56) References cited:
- WO-A1-2008/156780
- WO-A1-2008/156781

## Description

### Field of the Invention

This invention relates to a crystalline anhydrate and ansolvate form of the ethylenediamine salt of [4,6-bis(dimethylamino)-2-(4-{[4-(trifluoromethyl)benzoyl]amino}benzyl)pyrimidin-5-yl] acetic acid. The ethylenediamine salt of the invention is useful as an active ingredient in pharmaceutical preparations and can be used for the prophylaxis and treatment of diseases associated with CRTH2 activity.

### Background of the Invention

CRTH2 is a G protein-coupled chemoattractant receptor expressed on Th2 cells, eosinophils, and basophils (Nagata et al., J. Immunol. 1999, 162, 1278-1286; Hirai et al., J. Exp. Med. 2001, 193, 255-26 1). Prostaglandin D2 (PGD2), the major inflammatory mediator produced from mast cells, is a natural ligand for CRTH2. Recently, it has been shown that the activation of CRTH2 by PGD2 induces the migration and activation of Th2 cells and eosinophils, suggesting that CRTH2 may play a pro-inflammatory role in allergic diseases (Hirai et al., J. Exp. Med. 2001, 193, 255-261; Gervais et al., J. Allergy Clin. Immunol. 2001, 108, 982-988). It has also been shown that, in atopic dermatitis patients, there is an increase in circulating T cells expressing CRTH2, which correlates with the severity of the disease (Cosmi et al., Eur. J. Immunol. 2000, 30, 2972-2979; Iwazaki et al., J. Investigative Dermatology 2002, 119, 609-616). The role of PGD2 in the initiation and maintenance of allergic inflammation has further been demonstrated in mouse models of asthma by showing that overproduction of PGD2 in vivo by PGD2 synthase exacerbates airway inflammation (Fujitani et al., J. Immunol. 2002, 168, 443-449). Therefore, CRTH2 antagonists are potentially useful for the treatment of CRTH2-mediated disorders or diseases, such as allergic rhinitis, allergic asthma, bronchoconstriction, atopic dermatitis, or systemic inflammatory disorders. International Publication No. WO2008/15678 discloses the free-acid form of [4,6-bis(dimethylamino)-2-(4-{[4-(trifluoromethyl)benzoyl]amino}benzyl)pyrimidin-5-yl] acetic acid, which has the formula (I), 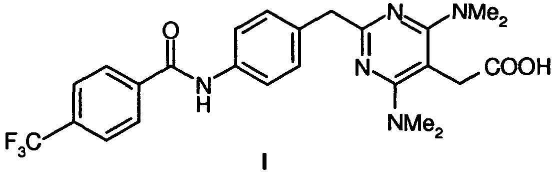 and reports that the compound is useful as a CRTH2 antagonist.

International Publication No. WO2008/156780 discloses two crystalline polymorphs of the free-acid form of the compound of formula (I).

International Publication No. WO2008/156781 discloses amine salts of the compound of formula (I) including a crystalline ethylenediamine salt in the form of an ethanol solvate.

Disclosed herein is a new polymorph of the ethylenediamine salt of the compound of formula (I) which is anhydrous, essentially free of organic solvent, and stable at elevated temperature.

### Summary of the Invention

In its broadest embodiment, the invention relates to a crystalline polymorph of an ethylenediamine salt of the compound of formula (I) which is anhydrous and non-solvated. The polymorphic salt of the invention contains about 2 molar equivalents of the compound of formula (I) per molar equivalent of ethylenediame. The polymorphic salt of the invention is further characterized by providing an X-ray powder diffraction pattern comprising 2θ angles of about 11.1, about 13.8, about 16.0, about 21.8, about 22.2, about 23.1, about 24.4, about 26.0, and about 26.5°. Applicants have found that this anhydrous and non-solvated form of the ethylenediamine salt of compound of formula (I) can exist in a novel crystalline polymorphic form which is referred to herein as "the Type II ethylenediamine salt" for convenience.

In another embodiment, the invention relates to a pharmaceutical composition comprising a pharmaceutically effective amount of the Type II ethylenediamine salt, at least one of a pharmaceutically acceptable carrier or excipient and, optionally, one or more further active compounds ("the pharmaceutical composition of the invention").

In another embodiment not covered by the claims, the invention relates to a method of treating or preventing one or more symptoms of a CRTH2-mediated disease or disorder comprising administering to a patient a therapeutically effective amount of the Type II ethylenediamine salt.

In another embodiment, the invention relates to a process of making the Type II ethylenediamine salt.

### Brief Description of the Drawings

FIG. 1 depicts an X-ray powder diffraction pattern of the Type II ethylenediamine salt of the invention.
FIG. 2 depicts an X-ray powder diffraction pattern of the Type I ethylenediamine salt.
FIG. 3 depicts X-ray powder diffraction patterns of samples of the Type II ethylenediamine salt (A-1) and the Type I ethylenediamine salt (B-1) which were both maintained at about 25°C and atmospheric pressure. FIG. 3 also depicts X-ray powder diffraction patterns of samples of the Type II ethylenediamine salt (A-2) and the Type I ethylenediamine salt (B-2) which were maintained at 80°C and at reduced pressure for about 15 hours.
FIG. 4 depicts a differential scanning calorimetric (DSC) and thermal gravimetric analysis (TGA) thermograms of the Type II ethylenediamine salt.
FIG. 5 depicts a differential scanning calorimetric (DSC) and thermal gravimetric analysis (TGA) thermograms of the Type I ethylenediamine salt.
FIG. 6 depicts a dynamic vapor sorption (DVS) isotherm plot of the Type II ethylenediamine salt.
FIG. 7 depicts a dynamic vapor sorption (DVS) isotherm plot of the Type I ethylenediamine salt.

### Detailed Description of the Inventions

As used herein, the term "free-acid" as it relates to the compound of formula (I) refers to non-salt forms of the compound of formula (I).

As used herein, the term "ansolvate" as it relates to the compound of formula (I) refers to forms of the compound of formula (I) which are essentially free of organic solvent (e.g., less than 1% by weight of organic solvent based on the total weight of compound of formula (I) including an counter-ions that may be present.

As noted above, Applicants have found a novel, crystalline form of the ethylenediamine salt of compound of formula (I) which is anhydrous and non-solvated (the Type II ethylenediamine salt). Characterization of the Type II ethylenediamine salt is provided below.

Applicants have found that the new Type II ethylenediamine salt of the invention exhibits higher thermal stability and is less hygroscopic than is an ethanolate form of the ethylenediamine salt of compound of formula (I) described in WO 2008/156781 and referred to herein as "the Type I ethylenediamine salt" for convenience.

The Type II ethylenediamine salt is further characterized by providing, in one embodiment, an X-ray powder diffraction pattern comprising 2θ angles of approximately 11.1, 13.8, 16.0, 21.8, 22.2, 23.1, 24.4, 26.0, and 26.5°.

In another embodiment, the Type II ethylenediamine salt is characterized by providing, an X-ray powder diffraction pattern comprising 2θ angles substantially similar to those shown in Table 1.

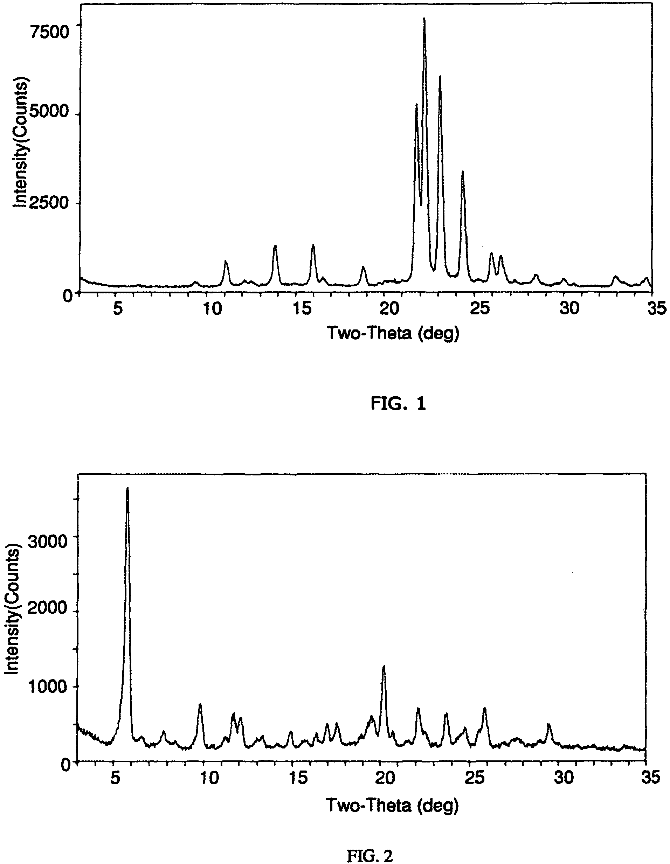

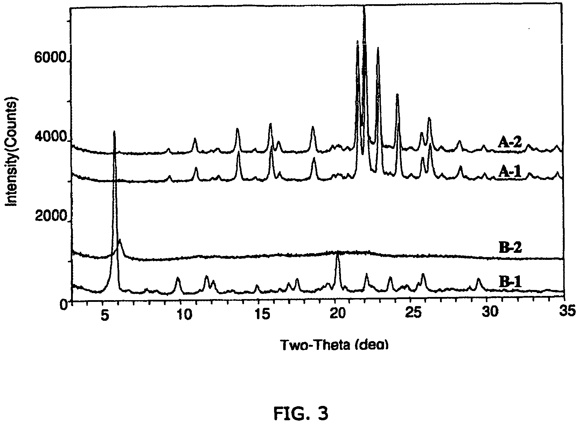

In another embodiment, the Type II ethylenediamine salt has an X-ray powder diffraction pattern substantially as shown in FIG. 1 and/or a differential scanning calorimetric thermogram as shown in FIG. 3.

In another embodiment, the Type II ethylenediamine salt has an X-ray powder diffraction pattern substantially as shown in FIG. 1.

In yet another embodiment, the Type II ethylenediamine salt has a DSC thermogram substantially as shown in FIG. 3.

In yet another embodiment, the Type II ethylenediamine salt has a DSC thermogram which exhibits an endotherm with a peak temperature of about 192°C and an onset temperature of about 160°C. In yet another embodiment, the Type II ethylenediamine salt has a DSC thermogram which exhibits an endotherm with a peak temperature of about 232°C and an onset temperature of about 225°C.

In yet another embodiment, the Type II ethylenediamine salt has a TGA thermogram which exhibits in one embodiment from about 5.5% to about 6.5% weight loss when heated between about 140°C to about 210°C; or from about 5.75% to about 6.25% weight loss when heated between about 140°C to about 210°C; or from about 5.75% to about 6% weight loss when heated between about 140°C to about 210°C

The Type II ethylenediamine salt can be prepared by the methods described immediately below and in the Examples.

In one embodiment, the invention relates to a process of making the Type II ethylenediamine salt comprising: (a) forming an admixture of an ethylenediamine salt of the compound of formula (I) in a suitable polar solvent ("the admixing step"); and (b) allowing the Type II ethylenediamine salt to crystallize from the admixture ("the crystallization step").

The ethylenediamine salt of the compound of formula (I) used in the admixing step immediately above can be a crystalline solvate and/or hydrate; an amorphous solvate and/or hydrate; an amorphous anhydrate; an amorphous ansolvate; or any combination of the foregoing.

Alternatively, the ethylenediamine salt of the compound formula (I) used in the admixing step above may be generated or formed in-situ by allowing a free-acid form of the compound of formula (I) and ethylenediamine to react in a suitable polar solvent to form the ethylenediamine salt of the compound formula (I). For the in-situ salt formation, the molar ratio of the free-acid form of the compound of formula (I) to ethylenediamine used for in-situ salt-formation can vary from about 3:1 to 1:3; from about 2.5:1 to about 2.25:1; or about 1:1. The resulting crystals of the ethylenediamine salt of the compound of formula (I) have a molar ratio of the free-acid form of the compound of formula (I) to ethylenediamine of about 2:1. The form of the compound of formula (I) used for in-situ salt-formation can be a solvate or hydrate of the free-acid form of the compound of formula (I), and can be amorphous or crystalline, e.g., the Form I described in WO2008156780. Alternatively, the free-acid compound of formula (I) used in the salt-forming step can be an amorphous anhydrate and/or ansolvate.

Thus, in another embodiment, the invention relates to a process of making the Type II ethylenediamine salt comprising: (a) combining a free-acid form of the compound of formula (I), ethylenediamine and a suitable polar solvent under conditions sufficient to form an admixture comprising an ethylenediamine salt of the compound of formula (I) (admixing step); and (b) allowing the Type II ethylenediamine salt to crystallize from the admixture (crystallization step).

As used herein, the term "suitable polar solvent" refers to an organic solvent that can dissolve at least a portion of the compound of formula (I), ethylenediamine, and the Type II ethylenediamine salt and will not form a coordination complex or solvate with the crystalline form of the compound of formula (I). Non-limiting examples of suitable polar solvents include acetonitrile, ethyl acetate, isopropyl acetate, and n-butyl acetate, and methyl isobutyl ketone. In one embodiment, the polar solvent is selected from the group consisting of acetonitrile, ethyl acetate, isopropyl acetate, methyl isobutyl ketone, and n-butyl acetate.

The admixing step in the embodiments described above is carried out for a time and at a temperature sufficient to allow at least a portion of the ethylenediamine salt of the compound of formula (I) to dissolve. In another embodiment, the admixing step in the embodiments described above is carried out for a time and at a temperature sufficient to allow at least a majority of the ethylenediamine salt of the compound of formula (I) is dissolved in the admixing step; and in another embodiment, essentially all of the ethylenediamine salt of the compound of formula (I) is dissolved in the admixing step.

A suitable temperature for the admixing step is from about 25°C to about the refluxing temperature of the polar solvent; in another embodiment, from about 25°C to about 40°C; in another embodiment, from about 40°C to about 65°C; and in another embodiment, about 40°C. A suitable time for the admixing step is typically from about 15 minutes to about 24 hours; or from about 15 minutes to about 5 hours; or from about 15 minutes to about 2 hours It will be understood that admixing step may include one or more temperature ramps including plateaus where the temperature may be held constant for a period of time.

The crystallization step in the embodiments described above is carried out for a time and at a temperature sufficient to allow at least a majority of ethylenediamine salt of the compound of formula (I) to crystallize or convert to the Type II ethylenediamine salt. A suitable temperature for the crystallization step is from about 25°C to about the refluxing temperature of the polar solvent; in another embodiment, from about 40°C to about the refluxing temperature of the polar solvent; in another embodiment, from about 40°C to about 65°C; in another embodiment, about 65°C; in another embodiment, about the refluxing temperature of the polar solvent; and in another embodiment, about 40°C. A suitable time for the crystallization step is typically from about 1 hour to about 72 hours; or from about 1 hour to about 48 hours; or from about 2 hours to about 24 hours. It will be understood that crystallization step may include one or more temperature ramps including plateaus where the temperature may be held constant for a period of time.

The embodiments for making the Type II ethylenediamine salt described above may include an additional step (c) comprising allowing the compositions of step (b) to further cool ("the cooling step") to a temperature that is lower than the temperature used in step (b). The temperature used in the optional cooling step is, in one embodiment, from about 0°C to about 40°C; in another embodiment, from about 15°C to about 40°C; and in another embodiment, from about 20°C to about 40°C. It will be understood that that the optional cooling step may include one or more temperature ramps including plateaus where the temperature may be held constant for a period of time.

In the embodiments described above, the process of making the Type II ethylenediamine salt typically further comprises the step of seeding the reaction admixture, prior to or during the initiation of the crystallization step. Thus, in one embodiment, the process for making the Type II ethylenediamine salt further comprises seeding the admixture during the admixing step or/and during the crystallization step. The seed crystals, when used, are a Type II ethylenediamine salt.

After crystallization and optional cooling step, the Type II ethylenediamine salt is separated from the liquid phase. Non-limiting methods of separation include filtration and decantation. Once separated, the Type II ethylenediamine salt can be washed with polar solvent and dried.

Characterization: As discussed above, the Type II ethylenediamine salt of the invention can be characterized by its X-ray powder diffraction pattern (XRPD) as shown in TABLE 1 and FIG. 1.

**TABLE 1. The relative intensity (%) and line spacing (d) of the characteristic reflection peaks (2θ) of the Type II ethylenediamine salt.**

| **Angle 2θ, °*** | **d, Å**** | **Relative Intensity, %** |
|---|---|---|
| 9.4 | 9.4 | 1.8 |
| 11.1 | 8.0 | 9.3 |
| 12.1 | 7.3 | 1.9 |
| 12.5 | 7.1 | 1.8 |
| 13.8 | 6.4 | 14.8 |
| 16.0 | 5.5 | 15.0 |
| 16.5 | 5.4 | 2.9 |
| 16.5 | 5.4 | 2.9 |
| 18.8 | 4.7 | 7.3 |
| 20.1 | 4.4 | 2.0 |
| 21.0 | 4.2 | 2.2 |
| 21.8 | 4.1 | 67.8 |
| 22.2 | 4.0 | 100.0 |
| 23.1 | 3.8 | 78.4 |
| 24.4 | 3.7 | 42.7 |
| 25.2 | 3.5 | 2.7 |
| 26.0 | 3.4 | 12.2 |
| 26.5 | 3.4 | 11.1 |
| 27.3 | 3.3 | 2.3 |
| 28.5 | 3.1 | 3.7 |
| 30.0 | 3.0 | 2.7 |
| 32.9 | 2.7 | 3.9 |
| 33.4 | 2.7 | 1.7 |
| 16.5 | 5.4 | 2.9 |

| | | |
|---|---|---|
| *The measured 2θ values have an uncertainty of ± 0.1°. **The d value is the spacing between the planes in the atomic lattice and is calculated from the measured 2θ value using Bragg's law: n λ =2d ·sin(θ), where n is an integer, and λ is the wavelength of radiation (here 1.54059Å). | | |

As discussed above, WO 2008/156781 describes an ethanolate form of the ethylenediamine salt of the compound formula (I) which is referred to herein as the Type I ethylenediamine salt. WO2008/156781 includes a thermogravimetric analysis of the ethanol solvate of the ethylenediamine salt of formula (I) which shows a slight weight loss from about 30°C to about 75°C and a larger weight loss from 125°C to 150°C which is attributed to loss of ethanol. The X-ray powder diffraction pattern of the Type I ethylenediamine salt is reported in TABLE 2 and shown in FIG. 2.

**TABLE 2. The relative intensity (%) and line spacing (d) of the characteristic reflection peaks (2θ) of the Type I ethylenediamine salt.**

| **Angle 2θ, °*** | **d, Å**** | **Relative Intensity, %** |
|---|---|---|
| 5.8 | 15.2 | 100.0 |
| 7.8 | 11.3 | 6.4 |
| 9.8 | 9.0 | 17.4 |
| 11.2 | 7.9 | 4.8 |
| 11.7 | 7.6 | 13.1 |
| 12.1 | 7.3 | 10.8 |
| 13.0 | 6.8 | 3.8 |
| 13.3 | 6.6 | 4.7 |
| 14.1 | 6.3 | 1.6 |
| 14.9 | 5.9 | 6.5 |
| 15.8 | 5.6 | 2.0 |
| 16.4 | 5.4 | 4.4 |
| 17.0 | 5.2 | 8.2 |
| 17.5 | 5.1 | 8.2 |
| 19.5 | 4.5 | 9.3 |
| 20.2 | 4.4 | 31.3 |
| 20.7 | 4.3 | 5.9 |
| 22.1 | 4.0 | 14.5 |
| 22.6 | 3.9 | 5.1 |
| 23.8 | 3.7 | 12.0 |
| 24.5 | 3.6 | 3.6 |
| 24.7 | 3.6 | 6.7 |
| 25.6 | 3.5 | 6.3 |
| 25.9 | 3.4 | 14.9 |
| 27.4 | 3.2 | 2.7 |
| 29.4 | 3.0 | 8.9 |
| 33.8 | 2.7 | 100.0 |

| | | |
|---|---|---|
| *The measured 2θ values have an uncertainty of ± 0.1°. **The d value is the spacing between the planes in the atomic lattice and is calculated from the measured 2θ value using Bragg's law: n λ =2d ·sin(θ), where n is an integer, and λ is the wavelength of radiation (here 1.54059Å). | | |

The XRPD reflections exhibited by the Type II ethylenediamine salt (TABLE 1 and FIG. 1) are different from those exhibited by the Type I ethylenediamine salt (TABLE 2 and FIG. 2). The results indicate that the Type II ethylenediamine salt is a different polymorphic form than the Type I ethylenediamine salt described herein and in WO 2008/156781.

Applicants have found that that the Type II ethylenediamine salt of the invention is more thermally stable and less hygroscopic than the Type I ethylenediamine salt as discussed below.

Effect of heat-treatment on crystallinity: Applicants carried out an XRPD analysis of samples of the Type I and Type II ethylenediamine salts exposed to elevated temperature (80°C), and the results are shown in FIG. 3. In FIG. 3, the top pattern A-1 was obtained on a sample of the Type II ethylenediamine salt maintained at atmospheric pressure and at about 25° prior to measuring the XRPD reflections. The pattern A-2 was obtained from a sample of the Type II ethylenediamine salt which was heated at 80°C under reduced pressure for 15 hours prior to measuring the XRPD reflections. As shown in FIG. 3, there was no noticeable change in the crystallinity of the Type II ethylenediamine salt as a result of heat treatment at 80°. The results indicate that the Type II ethylenediamine salt maintains its crystallinity at a temperature of at least about 80°C.

The bottom pattern B-1 was obtained on a sample of the Type I ethylenediamine salt which was maintained at atmospheric pressure and at about 25° prior to measuring the XRPD reflections. The pattern B-2 was obtained from a sample of the Type I ethylenediamine salt which was heated at 80°C under reduced pressure for 15 hours prior to measuring the XRPD reflections. As shown in FIG. 3, there was a significant change in the crystallinity of the salt. The result indicates that the Type I ethylenediamine salt undergoes a loss in crystallinity at elevated temperature (e.g., 80°).

The results of the XRPD study indicate that the Type II ethylenediamine salt is more thermally stable than the Type I ethylenediamine salt.

Weight loss at elevated temperature: Differences in thermal characteristics between the Type I and Type II ethylenediamine salts were also observed using differential scanning calorimetry (DSC), thermal gravimetric analysis (TGA) and dynamic vapor sorption (DVS) studies. TABLE 3 shows characteristic thermal events (DSC and TGA) for each of the Type I and Type II ethylenediamine salt polymorphs.

**Table 3 Thermal events of Type I and Type II ethylenediamine salts in DSC/TGA**

| **Polymoprh** | **Onset of event, °C** | **Comments** |
|---|---|---|
| Type I (ethanolate/hydrated) | 30 | Loss of water |
| | 120 | Melting and loss of ethanol and ethylenediamine |
| | 210 | Melting of the free-acid of compound of formula (I) |
| Type II (anhydrate, non-solvated) | 160 | Melting and loss of ethylenediame |
| | 225 | Melting of the free-acid of compound of formula (I) |

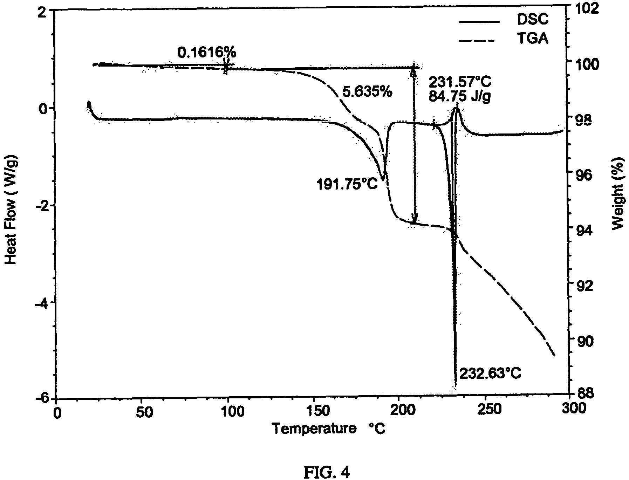

DSC and TGA thermograms of the Type II ethylenediamine salt (FIG. 4) indicate minimal loss of residual solvent up to 100°C (about 0.16% which is controllable by more thorough drying). The DSC thermogram shows two endothermic events. A first endothermic peak appears at about 192°C with about 5.6% of total weight loss in TGA at corresponding temperature region (140°C to 210°C). This weight loss corresponds roughly to the theoretical weight loss (5.99 wt%) of ethylenediamine from the ethylenediamine salt of the compound of formula (I) (which contains 0.50 molar equivalents of ethylenediamine). The result suggests that the first endothermic event is associated with the dissociation/loss of ethylenediamine. The following endothermic peak is shown at 232°C which is similar to the melting point of the free-acid form of the compound of formula (I).

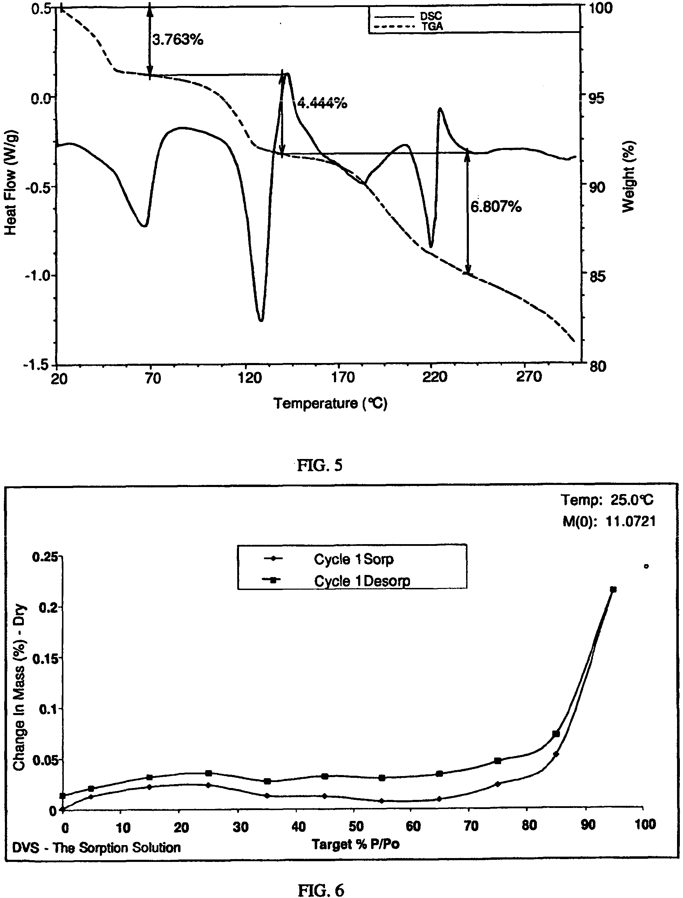

The DSC and TGA thermograms of the Type I ethylenediamine salt (FIG. 5) show unstable thermal behavior. There is about 3.8% weight loss at 20°C to 70°C, which corresponds to the loss of water. A major endotherm at 118°C to 134°C corresponds to the melting of the ethylenediamine salt with volatilization, corresponding to the loss of ethanol and some ethylenediamine (about 4.4%). Further loss of ethylenediamine occurs at about 147°C to 160°C. Above 165°C, melting and decomposition of the free-acid of the compound of formula (I) take place.

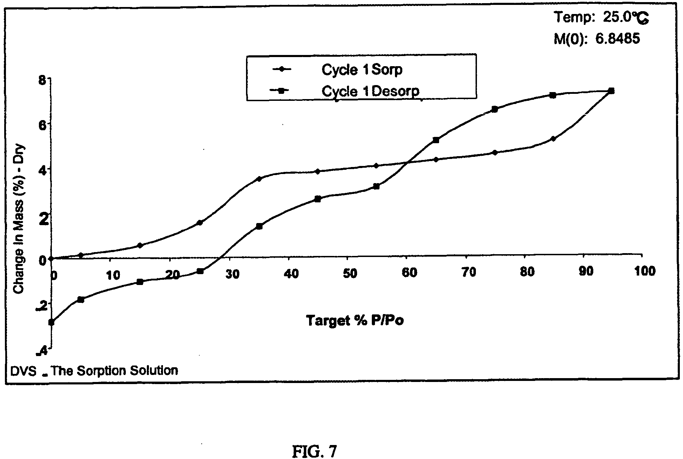

Water sorption/desorption profiles: Water sorption/desorption profiles of the Type II ethylenediamine salt are shown in FIG. 6. The Type II ethylenediamine salt exhibits about 0.08% of moisture gain through 85% relative humidity at 25°C indicating non-hygroscopic properties at the conditions used in this analysis. A similar water sorption/desorption study was carried out on the Type I ethylenediamine salt (FIG. 7). In the case of the Type I salt, significant moisture sorption was observed: ~6% water uptake at 70% relative humidity; and ~7% water uptake at 85% relative humidity. The sorption of water caused a form change in the Type I ethylenediamine salt which was observed using XRPD analysis.

The results of these studies show that the Type II ethylenediamine salt is less prone to hydration than is the Type I ethylenediamine salt.

### Pharmaceutical compositions

The pharmaceutical composition of the invention may be prepared in a form suitable for inhalative, oral, intravenous, topical, subcutaneous, intramuscular, intraperitoneal, intranasal, transdermal or rectal administration.

### A) Oral formulations

In one embodiment, the invention relates to a pharmaceutical composition of the invention that is suitable for oral administration comprising the Type II ethylenediamine salt and one or more of a pharmaceutically acceptable carrier or excipient

In another embodiment, the invention relates to a pharmaceutical composition that is suitable for oral administration consisting essentially of the Type II ethylenediamine salt.

Non-limiting examples of oral formulations include tablets, coated tablets, pills, granules or granular powder, syrups, emulsions, suspensions, or solutions, optionally together with inert and non-toxic pharmaceutically acceptable excipients or solvents

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups containing the active substances or combinations thereof according to the invention may additionally contain a sweetener such as saccharine, cyclamate, glycerol or sugar and a flavor enhancer, e.g., a flavoring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Capsules containing one or more active substances or combinations of active substances may for example be prepared by mixing the active substances with inert carriers such as lactose or sorbitol and packing them into gelatine capsules.

Carriers or excipients which may be used include, for example, water, pharmaceutically acceptable organic solvents such as paraffins (e.g., petroleum fractions), vegetable oils (e.g., groundnut or sesame oil), mono- or polyfunctional alcohols (e.g., ethanol or glycerol), carriers such as, e.g., natural mineral powders (e.g., kaolins, clays, talc, chalk), synthetic mineral powders (e.g., highly dispersed silicic acid and silicates), sugars (e.g., cane sugar, lactose and glucose), emulsifiers (e.g., lignin, spent sulphite liquors, methylcellulose, starch and polyvinylpyrrolidone) and lubricants (e.g., magnesium stearate, talc, stearic acid and sodium lauryl sulphate).

Tablets may additionally contain additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process.

Aqueous suspensions may be combined with various flavour enhancers or colourings in addition to the excipients mentioned above.

It will be understood that each of the oral formulations containing the Type II ethylenediamine salt may optionally contain one or more further active compounds as described below.

### B) Inhalative formulations

In one embodiment, the invention relates to a pharmaceutical composition suitable for inhalation comprising the Type II ethylenediamine salt and one or more of a pharmaceutically acceptable carrier or excipient.

In another embodiment, the invention relates to pharmaceutical composition suitable for inhalation consisting essentially of the Type II ethylenediamine salt and at least one of a pharmaceutically carrier or excipient.

Non-limiting examples of preparations suitable for inhalation include inhalable powders, propellant-containing metered-dose aerosols and propellant-free inhalable solutions. The inhalative formulations may optionally include inert and non-toxic pharmaceutically acceptable excipients or solvents as described below.

### B.1) Powder formulations:

The pharmaceutical composition of the invention can, in one embodiment, be in the form of an inhalable powder, optionally comprising pharmaceutically acceptable excipients.

Non-limiting examples of pharmaceutically acceptable excipients useful for powder formulations include monosaccharides (e.g., glucose or arabinose), disaccharides (e.g., lactose, saccharose, maltose, trehalose), oligo- and polysaccharides (e.g., dextran), polyalcohols (e.g., sorbitol, mannitol, xylitol), cyclodextrines (e.g., α-cyclodextrine, β-cyclodextrine, χ-cyclodextrine, methyl-β-cyclodextrine, hydroxypropyl-β-cyclodextrine), salts (e.g., sodium chloride, calcium carbonate) or mixtures of these excipients with one another. Preferably, mono- or disaccharides are used, while the use of lactose, trehalose or glucose is preferred, particularly, but not exclusively, in the form of their hydrates.

Within the scope of the inhalable powders according to the invention the excipients have in one embodiment a maximum average particle size of up to about 250µm; in another embodiment, from about 10 to about 250µm; in another embodiment, from about 10 to about 150µm; and in another embodiment, from about 15 to about 80µm.

The inhalable powders may further comprise finer excipient fractions with an average particle size of 1 to 9µm to the excipient mentioned above. These finer excipients are also selected from the group of possible excipients listed above. In order to prepare the inhalable powders according to the invention, a micronised form of the Type II ethylenediamine salt (and the one or more further active compounds when present), preferably with an average particle size of 0.5 to 10µm, more preferably from 1 to 6µm, is added to the excipient mixture. Processes for producing the inhalable powders according to the invention by grinding and micronising and by finally mixing the ingredients together are known from the prior art.

In one embodiment, the invention relates to a pharmaceutical composition in the form of an inhalable powder which contains only the Type II ethylenediamine salt as its active ingredient.

The inhalable powders according to the invention may be administered using inhalers known from the prior art. Inhalable powders according to the invention which contain one or more physiologically acceptable excipients may be administered, for example, by means of inhalers which deliver a single dose from a supply using a measuring chamber as described in US 4570630A, or by other means as described in DE 36 25 685 A. The inhalable powders according to the invention which contain the Type II ethylenediamine salt optionally in conjunction with a physiologically acceptable excipient may be administered, for example, using the inhaler known by the name Turbuhaler^{®} or using inhalers as disclosed for example in EP 237507 A. Preferably, the inhalable powders according to the invention which contain a physiologically acceptable excipient are packed into capsules (to produce so-called inhalettes) which are used in inhalers as described, for example, in WO 94/28958. A particularly preferred inhaler for using the inhalable powders according to the invention is the inhaler known by the name Handyhale®.

If the inhalable powders according to the invention are packed into capsules (inhalers) for the preferred use described above, the quantities packed into each capsule should be 1 to 30mg per capsule.

### B.2) Propellant-Containing Inhalable Aerosol

In another embodiment, the invention relates to a pharmaceutical composition in the form of a propellant-containing inhalable aerosol. Such formulations comprise the Type II ethylenediamine salt, and optionally one or more further active compounds, in dissolved and/or dispersed form.

Non-limiting examples of propellant gases useful in the propellant-containing inhalable aerosol include hydrocarbons such as n-propane, n-butane or isobutene; or halohydrocarbons such as chlorinated and/or fluorinated derivatives of methane, ethane, propane, butane, cyclopropane or cyclobutane.

In another embodiment, the propellant used in the propellant-containing inhalable aerosol is TG11 (trichlorofluoromethane), TG12 (dichlorodifluoromethane), TG134a (1,1,1,2-tetrafluoroethane), TG227 (1,1,1,2,3,3,3-heptafluoropropane), or mixtures thereof. In another embodiment, the propellant is TG 134a, TG227 or a mixture thereof.

The propellant-containing inhalable aerosols according to the invention may also contain other ingredients such as co-solvents, stabilisers, surfactants, antioxidants, lubricants and pH adjusters. All these ingredients are known in the art.

The propellant-containing inhalable aerosol according to the invention may contain up to 5 wt.% of the Type II ethylenediamine salt and, optionally, one or more further active compounds. Aerosols according to the invention contain, for example, 0.002 to 5 wt.%, 0.01 to 3 wt.%, 0.015 to 2 wt.%, 0.1 to 2 wt.%, 0.5 to 2 wt.% or 0.5 to 1 wt.% of the Type II ethylenediamine salt and the optional further active compounds.

If the Type II ethylenediamine salt and optional further active compounds are present in dispersed form, the particles of active substances have, in one embodiment, an average particle size of up to about 10µm; in another embodiment from about 0.1 to about 6µm; and in another embodiment, from about 1 to about 5µm.

The propellant-driven inhalation aerosols according to the invention may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterized in that they contain the propellant gas-containing aerosols described above according to the invention. The present invention also relates to cartridges fitted with a suitable valve which can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### B.3. Propellant-Free Inhalable Aerosols

In another embodiment, the invention relates to a pharmaceutical composition in the form of a propellant-free inhalable aerosol.

The propellant-free inhalable aerosol of the invention is in the form of a solution or suspension. Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but preferably the maximum is up to 70 percent by volume, more particularly up to 60 percent by volume of ethanol. The remainder of the volume is made up of water. The solutions or suspensions containing the Type II ethylenediamine salt and optional further active compound, separately or together, are adjusted to a pH of 2 to 7, preferably 2 to 5, using suitable acids. The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g., as flavorings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabilizer or complexing agent is unnecessary in the present formulation. Other embodiments may contain this compound or these compounds. In a preferred embodiment the content based on sodium editate is less than 100mg/100ml, preferably less than 50mg/100 ml, more preferably less than 20mg/100 ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g., alcohols, particularly isopropyl alcohol, glycols, particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavorings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

In one embodiment, the propellant-free inhalable solution comprises water, the Type II ethylenediamine salt, and a preservative. In another embodiment, the propellant-free inhalable solution comprises water, the Type II ethylenediamine salt, and a preservative selected from benzalkonium chloride and sodium editate. In yet another embodiment, the propellant-free inhalable solution comprises water, the Type II ethylenediamine salt, and benzalkonium chloride. In yet another embodiment, the propellant-free inhalable solution comprises water, the Type II ethylenediamine salt, and a preservative which is not sodium editate.

The propellant-free inhalable solutions according to the invention can be administered using inhalers of the kind which are capable of nebulizing a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 20 and 30µL of active substance solution can be nebulized in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulizers (devices) described therein are known by the name Respimat®.

In one embodiment, the invention relate to a pharmaceutical composition in the form of an inhalable solution optionally containing other co-solvents and/or excipients.

In another embodiment, the invention relates to a pharmaceutical composition in the form of an inhalable solution comprising at least one co-solvent containing hydroxyl groups or other polar groups, e.g., alcohols, particularly isopropyl alcohol glycols, particularly propyleneglycol, polyethyleneglycol, polypropyleneglycol, glycolether, glycerol, polyoxyethylene alcohols; and polyoxyethylene fatty acid esters.

In yet another embodiment, the invention relates to pharmaceutical composition in the form of an inhalable solution containing excipients selected from surfactants, stabilisers, complexing agents, antioxidants and/or preservatives, flavourings, pharmacologically acceptable salts and/or vitamins.

When the propellant-free inhalable aerosols comprise a further active compound, the doses applicable for the combinations according to the invention are to be understood as referring to doses per single application. However, it will be understood that these do not exclude the possibility of administering the combinations according to the invention multiple times. Depending on the medical need patients may receive also multiple inhalative applications. For example, patients may receive the combinations according to the invention for instance two or three times (e.g., two or three puffs with a powder inhaler, an MDI etc.) in the morning of each treatment day. As the aforementioned dose examples are only to be understood as dose examples per single application (i.e., per puff) multiple application of the combinations according to the invention leads to multiple doses of the aforementioned examples. The application of the compositions according to the invention can be for instance once a day, or depending on the duration of action of the agents twice a day, or once every 2 or 3 days.

It will be understood that the aforementioned dosages are to be understood as examples of metered doses only, i.e., the aforementioned doses are not to be understood as the effective doses of the combinations according to the invention that do in fact reach the lung. It is clear for the person of ordinary skill in the art that the delivered dose to the lung is generally lower than the metered dose of the administered active ingredients.

### The unit dose form and methods of administration

As noted above, the pharmaceutical composition of the invention may be administered in the form of a preparation suitable for inhalative, oral, intravenous, topical, subcutaneous, intramuscular, intraperitoneal, intranasal, transdermal or rectal administration. The pharmaceutical composition of the invention is applied to the patient as a unit dose form.

As used herein, the phrase "unit dose form" refers to the actual product, through which the pharmaceutical composition of the invention is administered to the patient. Non-limiting examples of unit dose forms include tablets, lozenges, capsules, inhalation powder capsules, unit dose vials, metered doses provided by a metered dose inhaler (MDI), injection vials and others commonly known by the skilled artisan.

In one embodiment, the invention relates to a method of orally administering the pharmaceutical composition to a patient in need thereof. Oral administration can be done one or more times per day in order to achieve the daily dosage for the patient. In another embodiment, the Type II ethylenediamine salt is administered orally twice a day. In another embodiment, the Type II ethylenediamine salt is administered orally once a day.

In another embodiment, the invention relates to an inhalative method for administering the pharmaceutical composition to a patient in need thereof. In yet another embodiment, the inhalative method comprises a pharmaceutical compositions selected from inhalable powders, propellant-containing metered-dose aerosols and propellant-free inhalable solutions. In another embodiment, the inhalative the inhalative method comprises an inhalable powder. In another embodiment, the inhalative the inhalative method comprises a propellant-containing metered-dose aerosol. And in another embodiment, the inhalative the inhalative method comprises a propellant-free inhalable solution.

In another embodiment, the invention relates to the use of a suppository to administer the pharmaceutical composition to a patient in need thereof. Suitable suppositories may be made for example by mixing with carriers provided for this purpose, such as neutral fats or polyethyleneglycol or the derivatives thereof.

The pharmaceutical composition of the invention can be applied to the patient via the unit dose form in one administration or in more than one sub-administration. In one embodiment, the daily dosages mentioned herein above are administered to the patient in a three-times-daily (t-d) administration scheme; in another embodiment, the daily dosages mentioned herein above are administered to the patient in a twice-daily (b-i-d) administration scheme; and in another embodiment, the daily dosages mentioned herein above are administered to the patient in a once-daily (q-d) administration scheme.

In one embodiment, the unit dose form comprises the Type II ethylenediamine salt in an amount of from about 1 mg to about 1000 mg; in another embodiment, from about 5 mg to about 800 mg; in another embodiment, from about 10 mg to about 700 mg; in another embodiment, from about 15 mg to about 600 mg; in another embodiment, from about 20 mg to about 500 mg; and in another embodiment, from about 25 mg to about 400 mg.

### Medical indications

The Type II ethylenediamine salt shows excellent CRTH2 antagonistic activity. It is, therefore, suitable for the prophylaxis and treatment of diseases associated with CRTH2 activity. It has been found that the pharmaceutical compositions described herein have a beneficial effect in terms of bronchospasmolysis and reduction of inflammations in the airways; allergic diseases of the oro-naso pharynx, skin or the eyes; inflammatory diseases of the joints; and inflammatory bowel disease.

In one embodiment, the invention relates to the treatment of an indication (A) selected from:
diseases of the airways and lungs which are accompanied by increased or altered production of mucus and/or inflammatory and/or obstructive diseases of the airways such as acute bronchitis, chronic bronchitis, chronic obstructive bronchitis (COPD), cough, pulmonary emphysema;
allergic or non-allergic rhinitis or sinusitis, chronic sinusitis or rhinitis;
nasal polyposis, chronic rhinosinusitis, acute rhinosinusitis;
asthma, allergic bronchitis, alveolitis, Farmer's disease, hyper-reactive airways;
bronchitis or pneumonitis caused by infection, e.g., by bacteria or viruses or helminthes or fungi or protozoons or other pathogens;
pediatric asthma, bronchiectasis;
pulmonary fibrosis;
adult respiratory distress syndrome, bronchial and pulmonary edema;
bronchitis or pneumonitis or interstitial pneumonitis caused by different origins e.g., aspiration, inhalation of toxic gases, vapors;
bronchitis or pneumonitis or interstitial pneumonitis caused by heart failure, X-rays, radiation, chemotherapy;
bronchitis or pneumonitis or interstitial pneumonitis associated with collagenosis, e.g., lupus erythematodes, systemic scleroderma;
lung fibrosis, idiopathic pulmonary lung fibrosis (IPF), interstitial lung diseases or interstitial pneumonitis of different origin, including asbestosis, silicosis, M. Boeck or sarcoidosis, granulomatosis;
cystic fibrosis or mucoviscidosis; or
α-1-antitrypsin deficiency.

Thus, in one embodiment, the invention relates to the use of a pharmaceutical composition of the invention for the manufacture of a medicament for treating respiratory diseases and conditions selected from indications (A) described above.

In another embodiment, the invention relates to a method of treating an indication selected from (A) above comprising administering a therapeutically effective amount of pharmaceutical composition of the invention to a patient in need thereof.

In yet another embodiment, the invention relates to a method of treating an indication (A) selected from chronic bronchitis, chronic obstructive bronchitis (COPD), chronic sinusitis, nasal polyposis, allergic rhinitis, chronic rhinosinusitis, acute rhinosinusitis, and asthma, the method comprising administering a therapeutically effective amount of pharmaceutical composition of the invention to a patient in need thereof.

In one embodiment, the invention relates to the treatment of an indication (B) selected from:
inflammatory diseases of the gastrointestinal tract of various origins such as inflammatory pseudopolyps, Crohn's disease, ulcerative colitis;
inflammatory diseases of the joints, such as rheumatoid arthritis; or
allergic inflammatory diseases of the oro-nasopharynx, skin or the eyes.

Thus, in one embodiment, the invention relates to the use of a pharmaceutical composition of the invention for the manufacture of a medicament for treating respiratory diseases and conditions selected from indications (B) described above.

In another embodiment, the invention relates to a method of treating an indication selected from indications (B) comprising administering a therapeutically effective amount of a pharmaceutical composition of the invention to a patient in need thereof.

In another embodiment, the invention relates to a method of treating an indication (B) selected from allergic inflammatory diseases of the oro-nasopharynx, skin or the eyes, Crohn's disease or ulcerative colitis.

In one embodiment, the present invention relates to a method for making a medicament for treating any of the aforementioned diseases and conditions by using a pharmaceutical composition of the invention, optionally containing one or more one further active compounds.

In another embodiment, the present invention relates to a method for making a medicament for treating asthma and allergic and non-allergic rhinitis by using a pharmaceutical composition comprising the Type II ethylenediamine salt, and optionally containing one or more further active compounds.

### Further active compounds

The pharmaceutical compositions of the invention can optionally comprise one or more additional active compound. Accordingly, in one embodiment, the invention relates to a pharmaceutical composition comprising a therapeutically effective amount of the Type II ethylenediamine salt, at least one of a pharmaceutically acceptable carrier or excipient, and at least one of a further active compound ("the combinations"). In another embedment, the invention relates to a method of administering the Type II ethylenediamine salt and at the least one of a further active compound to a patient in need thereof.

The actives of the combinations may be administered simultaneously, separately or sequentially. The preferred route of administration depends on the indication to be treated.

In one embodiment, the at least one further active compound is selected from the classes consisting of ß2-adrenoceptor-agonists (short and long-acting beta mimetics), anticholinergics (short and long-acting), anti-inflammatory steroids (oral and topical corticosteroids), dissociated-glucocorticoidmimetics, PDE3 inhibitors, PDE4- inhibitors, PDE7- inhibitors, LTD4 antagonists, EGFR- inhibitors, PAF antagonists, Lipoxin A4 derivatives, FPRL1 modulators, LTB4-receptor (BLT1, BLT2) antagonists, Histamine receptor antagonists, PI3-kinase inhibitors, inhibitors of non-receptor tyrosine kinases as for example LYN, LCK, SYK, ZAP-70, FYN, BTK or ITK, inhibitors of MAP kinases as for example p38, ERK1, ERK2, JNK1, JNK2, JNK3 or SAP, inhibitors of the NF-κB signalling pathway as for example IKK2 kinase inhibitors, iNOS inhibitors, MRP4 inhibitors, leukotriene biosynthese inhibitors as for example 5-Lipoxygenase (5-LO) inhibitors, cPLA2 inhibitors, Leukotriene A4 Hydrolase inhibitors or FLAP inhibitors, Non-steroidal anti-inflammatory agents (NSAIDs), DP1-receptor modulators, Thromboxane receptor antagonists, CCR1 antagonists, CCR2 antagonists, CCR3 antagonists, CCR4 antagonists, CCR5 antagonists, CCR6 antagonists, CCR7 antagonists, CCR8 antagonists, CCR9 antagonists, CCR10 antagonists, CXCR1 antagonists, CXCR2 antagonists, CXCR3 antagonists, CXCR4 antagonists, CXCR5 antagonists, CXCR6 antagonists, CX3CR1 antagonists, Neurokinin (NK1, NK2) antagonists, Sphingosine 1-Phosphate receptor modulators, Sphingosine 1 phosphate lyase inhibitors, Adenosine receptor modulators as for example A2a-agonists, modulators of purinergic receptors as for example P2X7 inhibitors, Histone Deacetylase (HDAC) activators, Bradykinin (BK1, BK2) antagonists, TACE inhibitors, PPAR gamma modulators, Rho-kinase inhibitors, interleukin 1-beta converting enzyme (ICE) inhibitors, Toll-Like receptor (TLR) modulators, HMG-CoA reductase inhibitors, VLA-4 antagonists, ICAM-1 inhibitors, SHIP agonists, GABAa receptor antagonist, ENaC-inhibitors, Melanocortin receptor (MC1R, MC2R, MC3R, MC4R, MC5R) modulators, CGRP antagonists, Endothelin antagonists, mucoregulators, immunotherapeutic agents, compounds against swelling of the airways, compounds against cough, CB2 agonists, retinoids, immunosuppressants, mast cell stabilizers, methylxanthine, opioid receptor agonists, laxatives, anti-foaming agents, antispasmodic agents, 5-HT4 agonists, and any combination thereof.

In another embodiment, the at least one further active compound is a PDE4 inhibitor. In yet another embodiment, the at least one further active compound is the PDE4 inhibitor Roflumilast.

In another embodiment, the at least one further active compound is a LTD4 antagonist. In yet another embodiment, the at least one further active compound is a LTD4 antagonist selected from montelukast, pranlukast and zafirlukast.

In another embodiment, the at least one further active compound is a histamine receptor antagonist. In yet another embodiment the at least one further active compound is a histamine receptor antagonist selected from azelastine, cetirizine, desloratidine, ebastine, epinastine, fexofenadine, hydroxyzine, ketotifen, levocetirizine, loratadine and olopatadine.

In another embodiment, the at least one further active compound is a 5-LO inhibitor. In yet another embodiment the at least one further active compound is the 5-LO inhibitor Zileuton.

In another embodiment, the at least one further active compound is a CCR5 antagonist. In yet another embodiment the at least one further active compound is the CCR5 antagonist Maraviroc.

In another embodiment, the at least one further active compound is a CCR9 antagonist. In yet another embodiment the at least one further active compound is the CCR9 antagonist Trafficet.

In another embodiment, the at least one further active compound is a Sulfonamide. In yet another embodiment the at least one further active compound is a Sulfonamide selected from Mesalazine and Sulfasalazine.

The Type II ethylenediamine salt and at least one of a further active compound may be combined in a single preparation, e.g., as a fixed dose combination comprising the active agents in one formulation together, or contained in two or more separate formulations, e.g., as a kit of parts adapted for simultaneous, separate or sequential administration. When the pharmaceutical compositions of the invention comprise one or more further active compounds, a single preparation is preferred.

When used in combination with a further active compound, the inhalable powders combination according to the invention may be prepared and administered either in the form of a single powder mixture which contains both the Type II ethylenediamine salt and the one or more further active compounds, or in the form of separate inhalable powders which comprise only the Type II ethylenediamine salt or the one or more further active compounds.

The daily dosage of the at least one further active compound, when present, is from about 1 mg to about 1000 mg; in another embodiment, from about 2 mg to 800 mg; in another embodiment, from about 3 mg to about 500 mg: in another embodiment, from about 4 mg to about 300 mg; in another embodiment, from about 5 mg to about 200 mg; and in another embodiment, from about 6 mg to about 150 mg.

### Experimental

The Type II ethylenediamine salts were characterized with nuclear magnetic resonance (NMR) spectroscopy, X-ray powder diffraction (XRPD), differential scanning calorimetry (DSC), thermogravimetric analysis (TGA), vapor sorption/desorption, and elemental analysis.

NMR spectra were recorded at 400 MHz on a Bruker NMR.

XRPD data were recorded with a Rigaku Miniflex II powder diffractometer (The Woodlands, Texas). The radiation was CuKa (30 kV, 15 mA). Data were collected at 25°C from 3 to 35 degrees 2θ at 0.02 degrees per step and 1.67 sec per step. Samples were prepared on Silicon (510) specimen holders as a thin layer of powdered material without solvent.

DSC was carried out using TA Instruments Q1000 Differential Scanning Calorimeter. Samples were placed in sealed aluminum pans for analysis with an empty aluminum pan as the reference. A heating rate of 10°C/min was employed over a temperature range from 20°C to 300°C.

TGA was carried out using TA Instruments Q500 Thermogravimetric analyzer. Samples were placed into an platinum sample pan. A heating rate of 10°C/min was employed over a temperature range from 25°C to 300°C.

Vapor sorption/desorption was carried out using Surface Measurement Systems DVS-HT. Samples were placed into a foil insert placed on a sample pan. Water sorption and desorption of the sample was observed at 25°C with stepwise change of relative humidity from 5% to 95% with two cycles of sorption/desorption. The equilibrium point of each step was reached when 0.002% of weight change was reached.

### Example 1

Seed crystals of the Type II ethylenediamine salt were prepared using the procedures described below. The seed crystals obtained in preparations (a) to (d) below were confirmed to be the Type II ethylenediamine salt XRPD (see FIG. 1 and TABLE 1).
(a) Ethylenediamine (93 uL) was dissolved in acetonitrile (1 mL) to provide a stock solution A portion of the stock solution (75 uL) was then added to a suspension of the free-acid of the compound of formula (I) (50 mg, 0.1 mmol) in acetonitrile (1 mL). The mixture was stirred at 40°C for 3 days and filtered. The solids were then washed with acetonitrile and air-dried to provide the Type II ethylenediamine salt (about 40 mg) as a white crystalline solid.
(b) A procedure similar to that described above in Example 1(a) was used except that ethyl acetate was used instead of acetonitrile to provide the Type II ethylenediamine salt (about 40 mg) as a white crystalline solid.
(c) A procedure similar to that described above in Example 1(a) was used except that isopropyl acetate was used instead of acetonitrile to provide the Type II ethylenediamine salt (about 40 mg) as a white crystalline solid.
(d) A procedure similar to that described above in Example 1(a) was used except that n-butyl acetate was used instead of acetonitrile to provide the Type II ethylenediamine salt (about 40 mg) as a white crystalline solid.

### Example 2

Preparatory-scale crystallizations of the Type II ethylenediamine salt were carried out using the seed crystals from Example 1 above using the procedures described below in parts (a) or (b).
(a) Ethylenediamine (93 uL) was dissolved in acetonitrile (1 mL) to provide a stock solution A portion of the stock solution (207 uL) was then added to a suspension of the free-acid form of the compound of formula (I) (0.152 g, 0.3 mmol) in acetonitrile (2 mL). The suspension was then treated with approximately 5 mg portion of the seed crystals of the Type II ethylenediamine salt obtained in Example 1 above. The mixture was heated to 65°C, stirred for 30 min, and cooled to 40°C. The mixture was agitated at 40° for 3 hours, cooled to 20°C, and filtered. The resultant solids were collected, washed with acetonitrile, and dried at 50°C under reduced pressure to provide the Type II ethylenediamine salt (0.137 g, 0.26 mmol) as white powdery crystals. Yield: 85.0%. Anal. Calcd for C₂₅H₂₆F₃N₅O₃ ·1/2 C₂H₈N₂ (non-solvated, anhydrous, 2:1 salt stoichiometry): C, 58.75%; H, 5.69%; N, 15.81%; F, 10.7%F. Found: C, 58.01%; H, 5.75%; N, 15.74%; F, 10.48% F. NMR analysis showed a 2:1 salt with 2.7 mole% residual acetonitrile due to incomplete drying (corresponding to 0.21 wt%).
   NMR analysis of the sample showed a 2:1 salt stoichiometry (molar basis) and minimal residual solvents (<0.21%). Elemental analysis is also consistent with the theoretical values for an anhydrous and non-solvated ethylenediamine salt of the compound of formula (I) having a 2:1 salt stoichiometry (molar basis).
   XRPD indicated that crystals were Type II ethylenediamine salt (see FIG. 1 and TABLE 1).
(b) Ethylenediamine (93 uL) was dissolved in ethyl acetate (1 mL) to provide a stock solution A portion of the stock solution (241 uL) was then added to a suspension of the free-acid form of the compound of formula (I) (0.177 g, 0.35 mmol) in ethyl acetate (10 mL). The suspension was then treated with approximately 5 mg portion of the seed crystals of the Type II ethylenediamine salt obtained in Example 1 above. The mixture was heated to 65°C, stirred at 65°C for 30 min, and stirred under reflux for 30 min. The mixture was cooled to 40°C, agitated for 15 hours at 40°C, and filtered. The resultant solids were then washed with ethyl acetate and dried at 70°C under reduced pressure for 15 hours to provide the Type II ethylenediamine salt (0.130 g, 0.24 mmol) as white powdery crystals. Yield: 69.9%. Anal. Calcd for C₂₅H₂₆F₃N₅O₃ · 1/2 C₂H₈N₂ (non-solvated, anhydrous, 2:1 salt stoichiometry): C, 58.75%; H, 5.69%; N, 15.81%; F, 10.7%F. Found: C, 57.95%; H, 5.76%; N, 15.61%; F, 10.81% F. NMR analysis showed a 2:1 salt with non-detectable residual ethyl acetate.
   XRPD indicated that crystals were Type II ethylenediamine salt (see FIG. 1 and TABLE 1).

### Example 3

A suspension of Type I ethylenediamine salt (ethanol solvate described above) (3.38 g, 6.36 mmol) in acetonitrile (50 mL) was treated with an approximately 20 mg portion of the seed crystals from Example 1 above. The mixture was heated to reflux and stirred for 30 min. The slurry was then cooled to 40°C, aged for 15 hours at 40°C, cooled to 20°C, and filtered. The solids were then washed with acetonitrile and dried for 15 hours at 80°C under reduced pressure to provided the Type II Ethylenediamine salt (3.04 g, 5.72 mmol) as white powder crystals. Yield: 89.9%. XRPD indicated that crystals were of the Type II ethylenediamine salt.

The examples set forth above are provided to give those of ordinary skill in the art with a complete disclosure and description of how to make and use the embodiments, and are not intended to limit the scope of the disclosure. Modifications of the above-described modes for carrying out the disclosure that are obvious to persons of skill in the art are intended to be within the scope of the inventions.

## Claims

1. A polymorphic form of an ethylenediamine salt of 4,6-bis(dimethylamino)-2-(4-{[4-(trifluoromethyl)benzoyl]amino}benzyl)pyrimidin-5-yl] acetic acid **characterized by** an X-ray powder diffraction pattern comprising 2θ angle values as shown below:
| **Angle 2θ, °** |
|---|
| about 11.1 |
| about 13.8 |
| about 16.0 |
| about 21.8 |
| about 22.2 |
| about 23.1 |
| about 24.4 |
| about 26.0 |
| about 26.5 |

2. The polymorph of claim 1 which provides an X-ray powder diffraction pattern substantially in accordance with that shown in Figure 1.

3. A pharmaceutical composition comprising a therapeutically effective amount of the polymorph of claim 1 and at least one of a pharmaceutically acceptable carrier or excipient.

4. The pharmaceutical composition of claim 3 which is suitable for oral administration, said pharmaceutical compositions selected from a tablet, coated tablet, pill, granule or granular powder, syrup, emulsion, suspension, and solution.

5. The pharmaceutical composition of claim 3 which is suitable for injectable administration.

6. The pharmaceutical composition of claim 3 which is suitable for inhalable administration, said pharmaceutical composition selected from inhalable powders, propellant-containing metered-dose aerosols and propellant-free inhalable solutions.

7. The polymorph of claim 1 for use in treating a disease associated with CRTH2 activity, the use comprising administering a therapeutically effective amount of the pharmaceutical composition of the invention to a patient in need thereof.

8. The polymorph of claim 1 for use in treating a disease selected from chronic bronchitis, chronic obstructive bronchitis (COPD), chronic sinusitis, nasal polyposis, allergic rhinitis, chronic rhinosinusitis, acute rhinosinusitis, and asthma, the use comprising administering a therapeutically effective amount of the pharmaceutical composition of the invention to a patient in need thereof.

9. A process for preparing the polymorph of claim 1 comprising:
(a) forming an admixture of an ethylenediamine salt of the compound of formula (I) in a suitable polar solvent; and
(b) allowing the Type II ethylenediamine salt to crystallize from the admixture.

10. A process for preparing the polymorph of claim 1 comprising:
(a) combining a free-acid form of the compound of formula (I), ethylenediamine and a suitable polar solvent under conditions sufficient to form an admixture comprising an ethylenediamine salt of the compound of formula (I); and
(b) allowing the Type II ethylenediamine salt to crystallize from the admixture.

11. The method of claim 9 or 10, further comprising seeding the admixture of step (a).

12. The method of any one of claims 9-11, wherein the polar, aprotic solvent is selected from acetonitrile, ethyl acetate, propyl acetate, and n-butyl acetates.

## Patentansprüche

1. Polymorphe Form eines Ethylendiaminsalzes von 4,6-Bis(dimethylamino)-2-(4-{[4-(trifluormethyl)benzoyl]amino}benzyl)pyrimidin-5-yl]essigsäure, **gekennzeichnet durch** ein Röntgenpulverdiffraktionsmuster, umfassend 2θ-Winkelwerte wie unten gezeigt:
| Winkel 2θ, ° |
|---|
| etwa 11,1 |
| etwa 13,8 |
| etwa 16,0 |
| etwa 21,8 |
| etwa 22,2 |
| etwa 23,1 |
| etwa 24,4 |
| etwa 26,0 |
| etwa 26,5 |

2. Polymorph nach Anspruch 1, der ein Röntgendiffraktionsmuster bereitstellt, das im Wesentlichen gemäß dem in Figur 1 gezeigten ist.

3. Pharmazeutische Zusammensetzung, umfassend eine therapeutisch wirksame Menge des Polymorphs nach Anspruch 1 und mindestens einen von: einem pharmazeutisch akzeptablen Träger oder Hilfsstoff.

4. Pharmazeutische Zusammensetzung nach Anspruch 3, die geeignet ist für die orale Verabreichung, wobei die pharmazeutischen Zusammensetzungen ausgewählt sind aus einer Tablette, einer beschichteten Tablette, einer Pille, einem Granulat oder einem granulären Pulver, einem Sirup, einer Emulsion, einer Suspension und einer Lösung.

5. Pharmazeutische Zusammensetzung nach Anspruch 3, die zur Verabreichung durch Injektion geeignet ist.

6. Pharmazeutische Zusammensetzung nach Anspruch 3, die zur Verabreichung durch Inhalation geeignet ist, wobei die pharmazeutische Zusammensetzung ausgewählt ist aus inhalierbaren Pulvern, Treibmittel-enthaltenden Dosier-Aerosolen und Treibmittel-freien inhalierbaren Lösungen.

7. Polymorph nach Anspruch 1 zur Verwendung bei der Behandlung einer Erkrankung, die mit CRTH2-Aktivität in Zusammenhang steht, wobei die Verwendung umfasst: die Verabreichung einer therapeutisch wirksamen Menge der pharmazeutischen Zusammensetzung der Erfindung an einen Patienten mit Bedarf hierfür.

8. Polymorph nach Anspruch 1 zur Verwendung bei der Behandlung einer Erkrankung, ausgewählt aus chronischer Bronchitis, chronischer obstruktiver Bronchitis (COPD), chronischer Sinusitis, nasaler Polypose, allergischer Rhinitis, chronischer Rhinosinusitis, akuter Rhinosinusitis und Asthma, wobei die Verwendung umfasst: die Verabreichung einer therapeutisch wirksamen Menge der pharmazeutischen Zusammensetzung der Erfindung an einen Patienten mit Bedarf hierfür.

9. Verfahren zur Herstellung des Polymorphs nach Anspruch 1, umfassend:
(a) Bilden einer Mischung eines Ethylendiaminsalzes der Verbindung der Formel (I) in einem geeigneten polaren Lösungsmittel; und
(b) Auskristallisieren lassen des Typ-II-Ethylendiaminsalzes aus der Mischung.

10. Verfahren zur Herstellung des Polymorphs nach Anspruch 1, umfassend:
(a) Kombinierten einer freien Säureform der Verbindung der Formel (I), Ethylendiamin und ein geeignetes polares Lösungsmittel unter Bedingungen, die ausreichend sind, um eine Mischung zu bilden, umfassend ein Ethylendiaminsalz der Verbindung der Formel (I); und
(b) Auskristallisieren lassen des Typ-II-Ethylendiaminsalzes aus der Mischung.

11. Verfahren nach Anspruch 9 oder 10, weiterhin umfassend das Impfen der Mischung von Schritt (a).

12. Verfahren nach irgendeinem der Ansprüche 9-11, wobei das polare aprotische Lösungsmittel ausgewählt ist aus Acetonitril, Ethylacetat, Propylacetat und n-Butylacetaten.

## Revendications

1. Forme polymorphe d'un sel d'éthylènediamine de l'acide 4,6-bis(diméthylamino)-2-(4-{[4-(trifluorométhyl)benzoyl]amino}benzyl)pyrimidin-5-yl]acétique **caractérisée par** un diagramme de diffraction des rayons x sur poudre comprenant des valeurs d'angle 2θ telles que présentées ci-dessous :
| Angle 2θ, ° |
|---|
| environ 11,1 |
| environ 13,8 |
| environ 16,0 |
| environ 21,8 |
| environ 22,2 |
| environ 23,1 |
| environ 24,4 |
| environ 26,0 |
| environ 26,5 |

2. Polymorphe selon la revendication 1, qui fournit un diagramme de diffraction des rayons X sur poudre
essentiellement conformément à celui représenté sur la figure 1.

3. Composition pharmaceutique comprenant une quantité thérapeutiquement efficace du polymorphe selon la revendication 1 et au moins l'un d'un support ou d'un excipient pharmaceutiquement acceptable.

4. Composition pharmaceutique selon la revendication 3, qui est appropriée pour une administration orale, lesdites compositions pharmaceutiques choisies parmi un comprimé, un comprimé enrobé, une pilule, des granulés ou une poudre granulaire, un sirop, une émulsion, une suspension, et une solution.

5. Composition pharmaceutique selon la revendication 3, qui est appropriée pour une administration injectable.

6. Composition pharmaceutique selon la revendication 3, qui est appropriée pour une administration inhalable, ladite composition pharmaceutique choisie parmi des poudres inhalables, des aérosols doseurs contenant un propulseur et des solutions inhalables dépourvues de propulseur.

7. Polymorphe selon la revendication 1, pour une utilisation dans le traitement d'une maladie associée à l'activité CRTH2, l'utilisation comprenant l'administration d'une quantité thérapeutiquement efficace de la composition pharmaceutique de l'invention à un patient en ayant besoin.

8. Polymorphe selon la revendication 1, pour une utilisation dans le traitement d'une maladie choisie parmi la bronchite chronique, une bronchopneumopathie chronique obstructive (BPCO), la sinusite chronique, la polypose nasale, la rhinite allergique, la rhinosinusite chronique, la rhinosinusite aiguë, et l'asthme, l'utilisation comprenant l'administration d'une quantité thérapeutiquement efficace de la composition pharmaceutique de l'invention à un patient en ayant besoin.

9. Procédé de préparation du polymorphe selon la revendication 1, comprenant :
(a) la formation d'un mélange d'un sel d'éthylènediamine du composé de formule (I) dans un solvant polaire approprié ; et
(b) le fait de laisser le sel d'éthylènediamine de type II cristalliser dans le mélange.

10. Procédé de préparation du polymorphe selon la revendication 1, comprenant :
(a) la combinaison d'une forme dépourvue d'acide du composé de formule (I), d'éthylènediamine et d'un solvant polaire approprié dans des conditions suffisantes pour former un mélange comprenant un sel d'éthylènediamine du composé de formule (I) ; et
(b) le fait de laisser le sel d'éthylènediamine de type II cristalliser dans le mélange.

11. Procédé selon la revendication 9 ou 10, comprenant en outre l'ensemencement du mélange de l'étape (a).

12. Procédé selon l'une quelconque des revendications 9 à 11, dans lequel le solvant aprotique polaire est choisi parmi l'acétonitrile, l'acétate d'éthyle, l'acétate de propyle, et les acétates de n-butyle.
